# FASCICULE DE BREVET EUROPEEN

(11) **EP 0 557 196 B1**
(45) Date de publication et mention de la délivrance du brevet: **03.01.1996**
(21) Numéro de dépôt: 93400409.4
(22) Date de dépôt: 18.02.1993
(51) Int. Cl.: A61K 7/00, A61K 7/032, A61K 7/48

(54) **Composition cosmétique pour le maquillage des yeux, comprenant une microdispersion de cire**
Make-up für die Augen, das eine Wachs-Mikrodispersion enthält
Eye make-up cosmetic composition containing a wax microdispersion

(30) Priorité: 21.02.1992 FR 9202059
(43) Date de publication de la demande: 25.08.1993
(73) Titulaire: L'OREAL, F-75008 Paris (FR)
(72) Inventeur: Piot, Bertrand, F-92250 La Garenne Colombes (FR); Mellul, Myriam, F-94240 l'Hay Les Roses (FR)
(74) Mandataire: Tonnellier, Jean-Claude

(56) Documents cités:
- EP-A- 0 394 078
- EP-A- 0 477 053
- WO-A-91/12793
- FR-A- 2 234 359
- GB-A- 2 216 797

## Description

La présente invention se rapporte à une composition de maquillage des yeux (cils et bord des paupières) dont les propriétés sont améliorées par la présence de cire à l'état microdispersé, associée à des polymères hydrosolubles et des pigments utilisés de façon classique dans ce type de composition.

Généralement, les compositions en vue du maquillage des cils, encore appelées "mascaras", ou des paupières (appelées ligneurs ou "eye-liners") sont constituées par des cires dispersées à l'aide d'un tensioactif dans une phase aqueuse contenant des polymères hydrosolubles et des pigments.

En choisissant qualitativement et quantitativement les cires et les polymères hydrosolubles, l'homme de l'art sait formuler des mascaras ayant des caractéristiques distinctes.

Ainsi, il est possible de réaliser diverses compositions qui, appliquées sur les cils, induisent des effets variés (allongement ou recourbement ou épaisssissement des cils). Il est en outre souhaitable d'obtenir un maquillage des cils dit "régulier", qui correspond à un état de surface lisse des cils après maquillage.

Il est difficile de concevoir un maquillage pour les cils à la fois épaississant et régulier, car l'effet épaississant est réalisé avec des compositions visqueuses et celles-ci sont incompatibles avec un dépôt lisse régulier.

Le document WO-A-91/12793 décrit une composition pour le revêtement des cils contenant, dans une solution aqueuse, une dispersion de cire, un polymère filmogène hydrosoluble et des pigments. Cette composition, qui est stable malgré l'absence d'agent émulsionnant, constitue un mascara dont la résistance à l'eau est améliorée.

Le document GB-A-2 216 797 décrit une composition stable de mascara contenant, sous forme d'une suspension ou émulsion aqueuse, une dispersion de cire, dont la caractéristique est de contenir des hydrolysats de kératine.

On a maintenant découvert que des compositions cosmétiques pour les cils renfermant des polymères hydrosolubles filmogènes, dans une microdispersion de cire contenant des pigments, induisent de façon surprenante des caractéristiques de maquillage très intéressantes. En effet, elles confèrent un bon effet d'épaississement des cils tout en donnant un maquillage très lisse et régulier, malgré une viscosité élevée de la composition, qui est nécessaire pour l'application sur les cils à l'aide d'une brosse.

Dans la demande de brevet européen EP-394 078, on a décrit l'utilisation comme composition cosmétique ou support de composition cosmétique pour cheveux, d'une composition fluide constituée essentiellement par une microdispersion stable de cires dans un véhicule liquide aqueux.

On a découvert que, de façon surprenante, après addition de polymères filmogènes et de pigments, des compositions à base de microdispersions de cire présentent des qualités remarquables de dépôt régulier et lisse sur les cils.

La présente invention a pour objet une composition cosmétique pour le maquillage des yeux, caractérisée par le fait qu'elle comprend une dispersion aqueuse de particules de cire, au moins un polymère filmogène et des pigments, et que ladite dispersion de cire est une microdispersion aqueuse d'au moins une cire.

Ladite composition a généralement une viscosité de 2,5 Pa.s à 35 Pa.s, comprise par exemple entre 3,5 et 25 Pa.s, à 25°C.

Les microdispersions de cire, qui sont des dispersions stables de particules colloïdales de cire, sont connues et peuvent être préparées selon des méthodes connues ; voir par exemple "Microemulsions Theory and Practice", L.M. Prince Ed., Academic Press (1977) pages 21-32.

Les particules de la microsdispersion de cire ont des dimensions inférieures à 1µm, de préférence inférieures à 0,5µm. Ces particules sont constituées essentiellement d'une cire ou d'un mélange de cires. Le point de fusion de la cire ou du mélange de cires est de préférence compris entre 50°C et 100°C. En outre, les particules de la microdispersion peuvent contenir en proportion minoritaire des additifs gras huileux ou pâteux, un ou plusieurs tensioactifs et un ou plusieurs ingrédients actifs liposolubles usuels, comme cela sera précisé ci-après.

La composition contient généralement de 0,1 à 40% en poids de cires, en particulier 5 à 30%, et une quantité suffisante d'au moins un émulsionnant. La quantité d'émulsionnant est une quantité suffisante pour permettre d'obtenir une microdispersion de cires telle que définie ci-dessus. Cette quantité suffisante peut être déterminée dans chaque cas par des expériences de routine.

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires constituant le mélange cireux sont choisies notamment, parmi la cire de Carnauba, la cire de Candellila, et la cire d'Alfa, et leurs mélanges.

Outre les cires citées ci-dessus, le mélange de cires peut également contenir une ou plusieurs des cires ou famille de cires suivantes :
- la cire de paraffine
- l'ozokérite
- les cires végétales comme la cire d'olivier, la cire de riz, la cire de jojoba hydrogénée ou les cires absolues de fleurs telles que la cire essentielle de fleur de cassis vendue par la Société BERTIN (France) ;
- les cires animales comme les cires d'abeilles, ou les cires d'abeilles modifiées (cerabellina) ;
- d'autres cires ou matières premières cireuses : les cires marines telles que celle vendue par la Société SOPHIM sous la référence M82, les céramides naturels ou de synthèse, ou les cires de polyéthylène.

Les cires végétales de Carnauba (extraite de Copernica Cerifera), de Candelilla (extraite de Euphobies Cerifera et de Pedilantus pavonis) et d'Alfa (extraite de Stipa tenacissima), sont des produits commerciaux.

Les céramides sont les principaux lipides constitutifs des espaces intercoméocytaires du Stratum Corneum. Ils sont décrits en particulier par Downing dans Science, 1982, vol.18, p.1261-2. Des analogues synthériques sont également connus, tels que les céramides HO3 vendus par la Société COSMIND.

Dans le mélange de cires, la cire de Carnauba et/ou de Candellila et/ou d'Alfa représente au moins 20%, et de préférence au moins 50% en poids par rapport au poids total du mélange de cires.

La cire ou le mélange de cires peut contenir, outre les cires mentionnées ci-dessus, au moins une autre cire et/ou au moins une huile, étant entendu que le mélange de cires et éventuellement d'huile à un point de fusion finissante supérieur à 50°C.

Le mélange de cires peut donc être associé à un ou plusieurs additifs gras (huileux ou pâteux). On citera de manière non restrictive :
- les huiles végétales comme l'huile de tournesol, l'huile de jojoba, etc. ;
- les huiles minérales comme l'huile de paraffine,
- les huiles de silicones fluides de viscosité comprise notemmant entre 0.65 et 100.000 centistokes (soit entre 0,65.10⁻⁴ et 10 m².s⁻¹, de préférence entre 5 et 5000 centistokes (soit entre 5.10⁻⁴ et 5.10⁻¹ m².s⁻¹),
- les huiles et cires fluorées,
- la vaseline,
- la lanoline.

Le mélange d'huile(s) et/ou d'additifs gras pâteux peut représenter jusqu'à 30% (de préférence au plus 10%) du poids de cires.

Il est possible d'introduire en outre dans la phase cireuse microparticulaire des ingrédients actifs liposolubles.

Lorsqu'ils sont présents, le ou les ingrédients liposolubles représentent au maximum 30%, de préférence au maximum 10%, du poids des microparticules.

Comme ingrédient(s) liposoluble(s), on citera par exemple :
- les filtres U.V.
- les vitamines liposolubles,
- les anti-inflammatoires tels que l'acide β-glycyrréthinique
- les extraits végétaux liposolubles.

L'utilisation de tensioactifs comme émulsionnants dans la préparation de microdispersions de cires est connue. La réalisation de la microdispersion peut être effectuée à l'aide de tensioactifs anioniques, cationiques et/ou non-ioniques, de façon connue.

Le pourcentage de tensio-actif(s) dans la composition finale est compris généralement entre 0.01 et 25% environ et en particulier peut varier de 0,1 à 10%.

Le rapport pondéral cire(s)/émulsionnant(s) peut varier par exemple dans la gamme 1 à 30 et notamment de 2 à 10.

Les tensioactifs anioniques utilisés sont notamment les sels d'acides gras (par exemple sels alcalins ou sels organiques tels que les sels d'amines) lesdits acides gras ayant par exemple de 12 à 16 atomes de carbone et pouvant comporter une double liaison comme dans le cas de l'acide oléique, les sels alcalins ou sels de bases organiques des acides alkyl-sulfuriques et alkylsulfoniques ayant 12 à 18 atomes de carbone, des acides alkyl-arylsulfoniques dont la chaîne alkyle contient de 6 à 18 atomes de carbone, le groupement aryle étant par exemple un groupement phényle. Ce sont également les éthers-sulfates, en particulier les produits de sulfatation des alcools gras et alkylphénols polyalcoxylés dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Tous ces tensioactifs anioniques sont bien connus et beaucoup d'entre eux sont des produits commerciaux.

Les tensio-actifs non ioniques sont principalement des tensio-actifs polyalcoxylés et/ou polyglycérolés. Ce sont notamment les acides gras ou les amides d'acide gras polyalcoxylés et/ou polyglycérolés ; les alcools gras ou les alkylphénols polyalcoxylés et/ou polyglycérolés ; les esters d'acides gras et de polyols polyalcoxylés et/ou polyglycérolés ; les alcanediols ou alcènediols-1,2 ou -1,3 polyalcoxylés et/ou polyglycérolés : et les alkyléthers d'alcanediols ou alcènediols-1,2 ou -1,3 polyalcoxylés et/ou polyglycérolés. Par exemple, les acides ou alcools gras, éventuellement insaturés, ont 12 à 24 atomes de carbone, la chaîne alkyle des alkylphénols a 6 à 16 atomes de carbone, les alcanediols ou alcènediols ont de 9 à 24 atomes de carbone, l'alkyle des alkyléthers a de 4 à 20 atomes de carbone, et le nombre de motifs oxyalkylène ou de motifs (CH₂CHOHCH₂O) peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la marque Myrj par la Société ATLAS.

Les esters d'acides gras et de polyols polyoxyéthylénés pour lesquels le polyol est le sorbitol sont des produits connus (Polysorbate et produits commercialisés sous la marque Tween par la Société ATLAS). Lorsque le polyol est le glycérol, on peut utiliser les produits commercialisés par le Société GOLDSCHMIDT sous la marque Tagat.

Les alcools gras polyoxyéthylénés sont des produits commerciaux, notamment ceux vendus sous la marque Brij par la Société ATLAS.

Les alcools gras polyglycérolés, les alcanediols ou alcanediols polyglycérolés, ou les alkyléthers d'alcanediols ou d'alcènediols polyglycérolés peuvent être préparés par exemple selon les procédés décrits dans les brevets français 1.477.048, 2.025.681, 2.091.516 et 2.465.780 ou selon des procédés analogues.

Les acides gras ou amides d'acides gras polyglycérolés sont notamment décrits dans le brevet français 1.484.723 ou sont encore des produits commerciaux tels que ceux vendus sous la marque PLUROL (Gattefossé) ou DREWPOL, (Stefan Company), ou DECAGLYN (NIKKO CHEMICAL).

D'autres tensioactifs non-ioniques utilisables sont par exemple :
- les alkylcarbamates de triglycérol de formule générale :

   R-NHCOOCH(CH₂OCH₂CHOHCH₂OH)₂

   dans laquelle R représente un groupe alkyle saturé ou non de 10 à 20 atomes de carbone. Ces composés sont décrits dans le brevet EP 0420761 ;
- les dérivés oxyéthylénés ou propoxylés des alcools de la lanoline, des acides gras de la lanoline, ou de leur mélanges.

De tels tensioactifs sont commercialisés par la Société AMERCHOL sous la marque SOLULAN.

Les tensioactifs cationiques sont notamment les dérivés d'ammonium quaternaire tels que l'ARQUAD 16-50, l'ARQUAD 18-50, l'ARQUAD T-50, l'ARQUAD 2C-75, l'ETHOQUAD c/12, et l'ETHOQUAD o/12, commercialisés par la Société Armak Chemicals.

L'emploi des tensioactifs non ioniques est préféré.

Il est également possible de préparer des microdispersions de cires en utilisant des mélanges commerciaux de cires auto-émulsionnables contenant la cire et les tensioactifs.

On peut utiliser par exemple la cire commercialisée sous la dénomination CIRE AUTO LUSTRANTE OFR par la Société TISCCO, qui contient des cires de Carnauba et de paraffine, en association avec des agents émulsionnants non ioniques, ou la cire auto-émulsionnable commercialisée sous la dénomination CERAX A.O. 28/B par LA CERESINE, qui contient de la cire d'Alfa en association avec un émulsionnant non ionique. Ces mélanges commerciaux permettant de préparer des microdispersions de cires par addition d'eau selon le procédé décrit ci-dessus.

On peut également utiliser des microdispersions de cires prêtes à l'emploi disponibles commercialement comme les produits de la série SL slipaid de la Société DANIEL PRODUCTS COMPANY, ou encore les produits Aquacer de la société CERACHEMIE.

Les microdispersions de cires sont diluables à l'eau sans nuire à la stabilité de la microdispersion. Elles peuvent donc se présenter sous la forme de compositions concentrées dont on peut ajuster la proportion des ingrédients à une valeur désirée par simple addition d'eau.

Les polymères filmogènes utilisables dans la composition de l'invention peuvent être des polymères anioniques, cationiques, non-ioniques ou amphotères.

Ces polymères filmogènes, de même que leur utilisation dans des compositions de maquillage pour les yeux, sont connus.

On décrit plus en détail ci-après à titre illustratif certains polymères filmogènes utilisables dans les compositions selon l'invention. On peut utiliser des polymères synthétiques ou des polymères d'origine naturelle, modifiés chimiquement ou non modifiés. On peut citer notamment les polymères cationiques qui sont des polymères du type polyamine, polyaminopolyamide ou polyammonium quaternaire dans lesquels le groupement amine ou ammonium fait partie de la chaîne polymère ou est relié à celle-ci ; ils ont un poids moléculaire généralement compris entre 500 et 3 000 000.

Comme polymères filmogènes cationiques utilisables, on peut citer par exemple :
1) les copolymères vinyl-pyrrolidone/acrylate ou méthacrylate de dialkylaminoalkyle (quaternisés ou non), tels que ceux vendus sous les dénominations "Gafquat" par la Société GAF CORP., comme par exemple le "copolymère 845" le "Gafquat 734 ou 735" décrits plus en détail dans les brevets FR 2 393 573 et FR 2 077 141.
2) les dérivés d'éthers de cellulose comportant des groupements ammonium quaternaires tels que ceux décrits dans le brevet FR 1 492 597 et notamment les polymères vendus sous les dénominations "JR" tels que "JR 125", "JR 400", "JR 30 M" et "LR", tels que "LR 400" et "LR 30 M" par la Société UNION CARBIDE CORP., les dérivés de cellulose cationiques tels que les "CELQUAT L 200" et "CELQUAT H 100", qui sont vendus par la Société NATIONAL STARCH ou ceux vendus par la Société AKZO sous la dénomination "LEOGARD GP".
3) les polysaccharides cationiques décrits dans les brevets US 3589978 et US 4031307 et en particulier le "Jaquar C13S"vendu par la Société MEYHALL.
4) les polymères cationiques contenant des motifs alternés de formule :

   -A-Z-

   dans laquelle A désigne un radical comportant deux fonctions amines et les groupements Z désignent au moins un radical bivalent, tels que les polymères décrits dans les brevets FR 2162025 et FR2280361.
5) les cyclopolymères tels que les homo- et copolymères de chlorure de diméthyl diallylammonium vendus sous la dénomination "Merquat" par la Société MERCK.
   Ces polymères sont décrits dans les brevets FR 2080759 et FR 2190406
6) les copolymères vinylimidazolium-vinylpyrrolidone quaternisés tels que ceux vendus sous le dénominations LUVIQUAT FC ou HM par la Société BASF.

Les polymères filmogènes anioniques sont des polymères comportant des groupes anioniques, en particulier carboxyliques et/ou sulfoniques.

Les polymères anioniques préférés utilisés dans les compositions de l'invention sont choisis notamment parmi :
1) les homo- ou copolymères d'acides acrylique ou méthacrylique ou leurs sels et leurs esters et en particulier les produits vendus sous les dénominations "VERSICOL F" ou "VERSICOL K" par la Société ALLIED COLLOID, "ULTRAHOLD 8" par la Société CIBA-GEIGY, les copolymères d'acide acrylique et d'acrylamide vendus sous la forme de leur sel de sodium sous les dénomination "Reten" par la Société HERCULES, le polyméthacrylate de sodium vendu sous la dénomination "Darvan n°7" par la société VANDERBILT, les sels de sodium d'acides polyhydroxycarboxyliques vendus sous la dénomination "HYDAGEN F" par la Société HENKEL.
2) les copolymères dérivés d'acide crotonique et leurs esters tels que ceux décrits par exemple dans les brevets FR 1222944, FR 1580545, FR 2265781, FR 2265782, FR 1564110 et FR 2439798.
3) les polymères ou copolymères dérivés d'acides ou d'anhydrides maléique, fumarique ou itaconique avec des dérivés vinyliques ou phénylvinyliques ou acryliques, ces polymères pouvant être estérifiés.
   De tels polymères sont décrits en particulier dans les brevets US 2047398, US.2723248, FR 2102113 et GB 839805.
   Parmi ces polymères, on peut citer les polymères vendus sous les dénominations "GANTREZ" par la Société GAF CORPORATION ou "Ema" par la Société MONSANTO. Des polymères entrant également dans cette classe sont les copolymères d'anhydrides maléique, citraconique ou itaconique et d'un ester allylique ou méthallylique comportant éventuellement un groupement acrylamide ou méthacrylamide dans leur chaîne, monoestérifiés ou monoamidifiés, décrits dans les brevets FR 2350834 et FR 2357241.
4) les polymères à groupements sulfonique utilisables conformément à l'invention sont choisis notamment parmi :
   - les sels de l'acide polystyrène sulfonique tels que ceux décrits notamment dans le brevet FR 2198719 ;
   - les sels de polyacrylamide sulfonique, tels que ceux mentionnés dans le brevet US 4128631 ;
   - les sels de polyesters tels que ceux vendus sous la dénomination "POLYMER EASTMAN AQ" par la Société KODAK ;
   - les kératines sulfoniques telles que celles décrites dans le brevet FR 2 529 214.

Les polymères filmogènes amphotères utilisables dans les compositions de l'invention sont notamment des polymères comportant des motifs M et M' répartis statistiquement dans la chaîne polymère, où M désigne au moins un motif dérivant d'un monomère comportant au moins un atome d'azote basique et M' désigne au moins un motif dérivant d'un monomère acide comportant un ou plusieurs groupements carboxyliques ou sulfoniques, ou bien M et M' peuvent désigner des groupements dérivant de monomères zwitérioniques de carboxybétaïne. M et M' peuvent également désigner une chaîne de polymère cationique comportant des groupements amine secondaire, tertiaire ou quaternaire, dans laquelle au mons l'un des groupements amino porte un groupement carboxylique ou sulfonique relié par l'intermédiaire d'un chainon hydrocarboné, ou bien M et M' font partie d'une chaîne d'un polymère à motif éthylène alpha-, bêta-dicarboxylique dont l'un des groupements carboxyliques a réagi avec une polyamine comportant un ou plusieurs groupement amine primaire ou secondaire.

On peut citer par exemple :
1) les polymères ou copolymères comportant des motifs dérivant :
   a) d'au moins un monomère choisi parmi les acrylamides ou les méthacrylamides, substitués à l'azote par un radical alkyle,
   b) d'au moins un monomère acide contenant un ou plusieurs groupements carboxyliques réactifs, et
   c) d'au moins un comonomère basique tels que les esters à substituant amine primaire, secondaire, tertiaire ou quaternaire des acides acrylique et méthacrylique et les produits de quaternisation de méthacrylate de diméthylaminoéthyle avec le sulfate de diméthyle ou de diéthyle ;
   Comme composés représentatif de cette classe on peut citer l'AMPHOMER" vendu par la société NATIONAL STARCH.
2) les polymères dérivés du chitosane tels que décrits par exemple dans le brevet FR 2137684 ou dans le brevet US 3879376 ;
3) les copolymères de diallyl dialkyl (C1-C4) ammonium/acide acrylique comme le produit vendu sous la dénomination "MERQUAT 280" par le Société MERCK qui est un copolymère de chlorure de diallyl diméthyl ammonium/acide acrylique.

Les polymères filmogènes non ioniques utilisables dans les compositions de l'invention sont notamment :
1) les homo- et/ou copolymères de vinylpyrrolidone tels que le copolymère polyvinylpyrrolidone/acétate de vinyle vendu sous la dénomination "PVP/PVA S-630" par la Société GAF, ou la dénomination "LUVISKOL" par la Société BASF ;
2) les homopolymères ou copolymères vinylique non ioniques tels que l'alcool polyvinylique vendu sous la dénomination "MOWIOL 4088" par la Société HOECHST
3) les poly-β-alanines décrites plus particulièrement dans la demande de brevet BE-20851 ;
4) les dérivés d'acide polyaspartique tels que ceux décrits dans le brevet FR.2403076 ;
5) ou encore les polyglycols tels que les polyéthylèneglycols ;
On peut citer aussi la famille des polyuréthanes en solution ou dispersion anionique, cationique ou non ionique, ainsi que la famille des protéines quaternisées ou non, parmi lesquelles les dérivés de kératine (tels que le "KERASOL" vendu par la Société CRODA).

Parmi les polymères filmogènes utilisables dans les compositions de l'invention, on peut citer aussi les polymères cellulosiques et saccharidiques comme l'hydroxyméthylcellulose, la carboxyméthylcellulose, l'hydroxybutylcellulose, l'hydroxypropylcellulose, et plus particulièrement l'hydroxyéthylcellulose, en particulier les produits vendus sous la dénomination "NATROSOL" par la Société HERCULES ou "CELLOZISE" par la Société UNION CARBIDE, la méthylhydroxypropyl cellulose, en particulier les produits vendus sous la dénomination "METHOCEL" par la Société DOW CHEMICAL ou des hétérobiopolysaccharides tels que par exemple les gommes de xanthane commercialisées sous les marques "KELTROL" et "KELZAN" par la Société KELCO, "RHODOPOL" et "RHODIGEL" par la Société RHONE POULENC, ou "ACTIGUM" par la Société CECA/SATIA, les gommes arabiques, la gomme de guar, la gomme de Karaya, les alginates et carraghenates, l'acide hyaluronique et ses dérivés.

Les polymères filmogènes sont choisis de préférence parmi l'hydroxyéthyl cellulose, la gomme arabique, la polyvinyl pyrrolidone, les dérivés de cellulose cationiques, le polyméthacrylate de sodium et les hydrolysats de kératine.

Les polymères filmogènes sont présent dans la composition généralement à raison de 0.1% à 25% en poids et de préférence de 0.2 à 15%.

La composition peut contenir un ou plusieurs polymères en solution dans la phase aqueuse de la préparation.

La composition selon l'invention contient au moins un pigment dans une proportion pouvant aller notamment jusqu'à 20% et de préférence entre 0.1 et 20% en poids par rapport au poids total de la composition, suivant la coloration et l'intensité de coloration que l'on cherche à obtenir. L'utilisation de pigments dans de telles compositions est connue en soi. La notion de pigment englobe des charges particulaires non colorées.

Les pigments utilisables sont choisis notamment permi les pigments minéraux, les pigments organiques, les pigments nacrés.

Parmi les pigments minéraux, on peut citer, à titre d'exemples :
- le dioxyde de titane (rutile ou anatase), éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome (CI 77288) ;
- l'hydrate de chrome (CI 77289) ; et
- le bleu ferrique (CI 77510).

Parmi les pigments organiques, on peut citer en particulier les pigments certifiés aux Etats-Unis d'Amérique par la FOOD & DRUG ADMINISTRATION sous les dénominations :

| | |
|---|---|
| - D & C red | n° 19 (CI 45170) ; |
| - D & C red | n° 9 (CI 15585) ; |
| - D & C red | n° 30 (CI 73360) ; |
| - D & C red | n° 3 (CI 45430) ; |
| - D & C red | n° 21 (CI 45380) ; |
| - D & C red | n° 27 (CI 45410) ; |
| - D & C red | n° 13 (CI 15630) ; |
| - D & C red | n° 7 (CI 15850-1) ; |
| - D & C red | n° 6 (CI 15850-2) ; |
| - D & C red | n° 36 (CI 12085) ; |
| - D & C orange | n° 10 (CI 45425) ; |
| - D & C orange | n° 4 (CI 15510) ; |
| - D & C orange | n° 5 (CI 45370) ; |
| - D & C yellow | n° 6 (CI 15985) ; |
| - D & C yellow | n° 5 (CI 19140) ; |

ainsi que :
- le noir de carbone (CI 77266) ; et
- la mélanine naturelle ou de synthèse ;
- les laques à base de carmin de cochenille (CI 75470) ;

Les pigments nacrés peuvent être choisis notamment parmi :
- les pigments nacrés blancs, tels que le mica recouvert d'oxyde de titane, l'oxychlorure de bismuth ; et le nitrure de bore ;
- les pigments nacrés colorés, tels que le mica titane avec des oxydes de fer, le mica titane avec du bleu ferrique, ou de l'oxyde de chrome, le mica titane avec un pigment organique du type précité, ainsi que ceux à base d'oxychlorure de bismuth ;
- les pigments enrobés tels que ceux obtenus à partir des pigments mentionnés ci-dessus et dont la surface a été traitée par diverses substances comme, par exemple, des acides aminés, des silicones, des sels métalliques ou du collagène.

En outre, la composition selon l'invention peut éventuellement contenir une ou plusieurs charges particulaires usuelles utilisées dans ce type de composition telles que : le talc qui est un silicate de magnésium hydraté, utilisé sous la forme de particules généralement de dimensions inférieures à 40 micromètres ; les micas, qui sont des aluminosilicates de compositions variées se présentant sous la forme d'écailles ayant généralement des dimensions de 2 à 200 micromètres, de préférence de 5 à 70 micromètres, et une épaisseur de 0.1 à 5 micromètres, de préférence de 0.2 à 3 micromètres. Ces micas peuvent être d'origine naturelle (par exemple muscovite, margarite, roscoelithe, lipidolithe, biotite) ou d'origine synthétique ; l'amidon, en particulier l'amidon de riz ; le kaolin, qui est un silicate d'aluminium hydraté et qui se présente sous la forme de particules de forme isotrope ayant des dimensions généralement inférieures à 30 micromètres ; les oxydes de zinc et de titane, généralement utilisés sous la forme de particules ayant des dimensions ne dépassant pas quelques micromètres ; le carbonate de calcium, le carbonate ou l'hydrocarbonate de magnésium ; la cellulose microcristalline ; les poudres de polymères synthétiques, tels que le polyéthylène, les polyesters (par exemple l'isophtalate ou le téréphtalate de polyéthylène), les polyamides (par exemple les poudres de nylon), les "téflons" et les poudres de silicone.

De façon générale, les charges colorées ou non colorées, peuvent être enrobées par des substances telles que des acides aminés, des silicones, des savons métalliques ou du collagène, ou avoir subi tout autre traitement permettant de modifier l'état de surface.

Les compositions cosmétiques selon l'invention peuvent aussi contenir un ou plusieurs adjuvants usuels tels que des agents épaississants, des parfums, des agents conservateurs, des agents alcalinisants ou acidifiants, des agents de textures, des additifs d'étalement, des plastifiants ainsi que des ingrédients actifs hydrosolubles utilisés habituellement dans les préparations pour les cils.

Dans les compositions de l'invention, les polymères filmogènes peuvent exercer un effet épaississant. Pour ajuster, le cas échéant, la viscosité désirée pour la composition, on peut ajouter des agents épaississants additionnels usuels.

L'agent ou les agents épaississants additionnels, lorsqu'ils sont présents, ont pour effet de stabiliser la dispersion des pigments et d'éviter ainsi la décantation de ces pigments, et donnnent à la composition finale la consistance nécessaire favorisant l'obtention d'un gainage avec épaississement du cil.

L'épaississement des formulations peut être aussi apporté par le polymère filmogène ou par l'association du polymère filmogène et d'un agent de texture choisi par exemple parmi les silices colloïdales, les bentones ou le distéarate de polyéthylène glycol comportant 150 motifs d'oxyde d'éthylène.

On peut utiliser d'autres agents épaississants connus qui peuvent être choisis par exemple parmi les acides polyacryliques réticulés par un agent polyfonctionnel tels que les produits vendus sous la dénomination CARBOPOL par la Société GOODRICH, comme les Carbopols 910, 934, 934P, 940, 941, 980, 1342, les argiles naturelles ou modifiées comme les Laponites de la Société LAPORTE ou Veegum de la Société VANDERBILT ou les polyuréthanes.

Avec ou sans épaississants additionnels, la viscosité des préparations selon l'invention est comprise entre 2,5 et 35 Pa.s et de préférence entre 3,5 à 25 Pa.s à 25°C environ, par exemple lorsqu'elle est mesurée au viscosimètre de Contraves (temps de rotation 10min à 200t/min)
Les additifs d'étalement peuvent être des dérivés fluorés comme ceux vendus par la Société 3M sous le nom "Fluorad", ou des tensioactifs diméthicone copolyol à HLB élevé disponible chez DOW CORNING, GOLDSCHMIDT, etc.

Comme plastifiants on pourra citer, par exemple, les PEG (polyéthylèneglycols) de faible masse moléculaire, la glycérine, le panthénol.

Les compositions selon l'invention sont obtenues par formation à chaud d'une microémulsion. Plus précisément, ces compositions sont obtenues par un prodédé principalement caractérisé par le fait que l'on chauffe la cire et l'émulsionnant à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microémulsion de cire dans une phase continue aqueuse, puis que l'on laisse refroidir jusqu'à la température ambiante. On obtient une microdispersion stable de cire.

On opère avec une agitation et une quantité de tensio-actif suffisantes pour que les dimensions des microparticules de cire soient inférieures à 1 000nm, et de préférence à 500nm.

Les ingrédients liposolubles, par exemple des céramides, sont généralement ajoutés à la cire avant la réalisation de la microdispersion.

Les ingrédients hydrosolubles peuvent être ajoutés dans l'eau utilisée pour réaliser la microdispersion, ou dans la microdispersion de cire finalement obtenue.

De même, les ingrédients secondaires éventuellement présents dans la composition sont ajoutés selon les cas soit dans les produits de départ, soit dans la composition terminée.

Les compositions de l'invention sont appliquées soit sur les cils, soit sur le bord des paupières, de façon comme, à l'aide d'une brosse ou d'un pinceau.

L'invention a également pour objet l'utilisation d'une microdispersion aqueuse de cire, en association avec au moins un polymère filmogène hydrosoluble et des pigments, dans la préparation d'une composition pour le maquillage des yeux.

La composition obtenue peut présenter les autres caractéristiques qui ont été décrites ci-dessus.

L'invention a également pour objet un procédé de maquillage des yeux, caractérisé par le fait que l'on applique sur les cils ou sur le bord des paupières une composition telle que définie précédemment.

Les exemples suivants illustrent l'invention.

### EXEMPLE DE PREPARATION DE MICRODISPERSIONS DE CIRES

### EXEMPLES A et B

| | A | B |
|---|---|---|
| .Cire de Carnauba | 40,0g | 30,0g |
| .Monostéarate de glycérol polyoxyéthyléné (300E) vendu sous la dénomination "TAGAT S" par la Société GOLDSCHMIDT | 10,0g | 7,5g |
| .Parahydroxybenzoate de méthyle | 0,2g | 0,2g |
| .Eau q.s.p. | 100,0g | 100,0g |

On porte à 90°C (en général 10°C au-dessus du point de fusion de la cire ou du mélange de cires et corps gras) le mélange des cire(s), conservateurs, tensioactif(s), en homogénéisant sous agitation modérée.

En continuant d'agiter, on incorpore l'eau portée à 90°C.

La microémulsion obtenue est ramenée à température ambiante et forme une microdispersion de grains à base de cire(s).

Diamètre moyen des particules de cire : A = 184nm B = 155nm

### EXEMPLE C

| | |
|---|---|
| .Cire de Carnauba | 30,0g |
| .Alcool de lanoline et mélange d'alcools gras polyoxyéthylénés (25 OE) vendu sous la dénomination "SOLULAN 25" par la Société AMERCHOL | 7,5g |
| .Parahydroxybenzoate de méthyle | 0,2g |
| .Eau q.s.p. | 100.0g |

La microdispersion est obtenue en procédant comme décrit dans les exemples A et B.

Diamètre moyen des particules de cire : 99nm
"x OE" signifie : oxyéthyléné à x moles d'oxyde d'éthylène.

### EXEMPLE D :

| | |
|---|---|
| .Cire de Carnauba | 22,5 g |
| .Cire de paraffine | 7,5 g |
| .Cire d'abeilles | 10,4 g |
| .Parahydroxybenzoate de méthyle | 0,2 g |
| .Monostéarate de glycérol polyoxyéthyléné (30 OE) vendu sous la dénomination "TAGAT S" par la Société GOLDSCHMIDT | 7,5 g |
| .Eau q.s.p. | 100,0 g |

La microdispersion est obtenue en procédant comme décrit dans les exemples A et B.

Dimension moyenne des particules de cire : 202nm

### EXEMPLE E

| | |
|---|---|
| .Cire de carnauba | 22,5 g |
| .1-(2'-F-hexyléthylthio)-3-(2''-éthylhexyloxy) propane-2-ol | 7,5 g |
| .Tagat S | 7,5g |
| .Parahydroxybenzoate de méthyle | 0,2 g |
| .Eau q.s.p. | 100 g |

La microdispersion est obtenue en procédant comme décrit dans les exemples A et B.

Diamètre moyen des particules de cire : 230 nm.

L'huile fluorée 1-(2'-F-hexyléthylthio) 3-(2''-éthylhexyloxy)-2-propane-2-ol est préparée de la façon suivante :

A la température de 25°C, sous agitation et sous courant d'azote, on ajoute à 152 g de 2-F-hexyléthanethiol, 3,6 g d'une solution méthanolique de méthylate de sodium (environ 30 % - 5,54 meq g⁻¹) en une minute.

Le mélange est chauffé à 70°C. On évapore sous vide le méthanol présent dans le milieu.

Du 2-éthylhexylglycidyléther (74,4 g) est ensuite ajouté goutte-à-goutte en une heure. On maintient la température du mélange entre 60 et 70 °C au cours de l'addition de l'époxyde.

A la fin de l'addition, la température est amenée à 25°C.

Le mélange est neutralisé à l'aide de 20 ml de HCl 1N.

Le 1-(2'-F-hexyléthylthio)3-(2''-éthylhexyloxy)-2-propanol est séparé par distillation : Eb = 141°C/66,5 Pa.

On obtient 175 g (77 %) d'une huile translucide incolore.

### EXEMPLE F

| | |
|---|---|
| .Cire de Carnauba | 18 g |
| .1-(2'-F-octyléthylthio)2-hexanol | 12 g |
| .Tagat S | 7,5 g |
| .Parahydroxybenzoate de méthyle | 0,2 g |
| .Eau q.s.p. | 100 g |

La microdispersion est obtenue en procédant comme décrit dans les exemples A et B.

Diamètre moyen des particules de cires : 195 nm.

La cire fluorée 1-(2'-F-octyléthylthio)2-hexanol est préparée de la façon suivante :

Selon un mode opératoire analogue à celui décrit à l'exemple E, on condense, en 1 heure, 30 g (0,3 mole) de 1,2-époxyhexane avec 144 g (0,3 mole) de 2-F-octyléthanethiol en présence de 2,7 g de solution méthanolique de méthylate de sodium (5,65 meq g⁻¹).

En fin de réaction, le mélange est neutralisé par 15 ml de HCl 1N.

Après distillation (154°C/133Pa), on obtient 115 g d'un solide blanc amorphe qui est le 1-(2'-F-octyléthylthio)2-hexanol.
Rendement = 67 %.
Point de fusion = 45°C.

### EXEMPLES DE COMPOSITION DE MAQUILLAGE POUR LES YEUX

On a préparé des compositions de maquillage pour les yeux en mélangeant aux microdispersions ci-dessus les ingrédients indiqués. Les teneurs sont exprimées en gramme.

### EXEMPLE 1

| | |
|---|---|
| .Microdispersion de cires selon l'exemple A | 93,75 |
| .Oxyde de fer noir | 6,00 |
| .Hydroxyéthyl cellulose vendue sous la dénomination "CELLOSIZE QP 4400 H" par la Société AMERCHOL | 0,25 |

### EXEMPLE 2

| | |
|---|---|
| .Microdispersion de cire selon l'exemple A | 89,7 |
| .Oxyde de fer noir | 6,0 |
| .Hydroxyéthylcellulose vendue sous la dénomination "CELLOSIZE QP 4400 H" par la société AMERCHOL | 1,0 |
| .Gomme arabique | 1,0 |
| .Eau | 2,3 |

### EXEMPLES 3, 4 et 5

| | 3 | 4 | 5 |
|---|---|---|---|
| .Microdispersion de cire selon l'exemple A | 50,0 | 89,0 | 87,7 |
| .Polyvinyl pyrrolidone vendue sous la dénomination "LUVISKOL K90 POWDER" par la Société BASF | 6,0 | 4,0 | 6,0 |
| .Oxyde de fer noir | 6,0 | 6,0 | 6,0 |
| .Gomme arabique | - | 1,0 | - |
| .Eau | 38,0 | - | - |
| .Tensioactif fluoré vendu sous la dénomination "FLUORAD FC 143" par la Société 3M | - | - | 0,3 |
| Brillance de la composition | 77 | 60 | 77 |

### EXEMPLES 6 et 7

| | 6 | 7 |
|---|---|---|
| .Cire de Carnauba | 15,8 | 23,7 |
| .Cire de paraffine | 1,7 | 2,6 |
| .Monostéarate de glycérol polyoxyéthyléné (30 OE) vendu sous la dénomination "TAGAT S" par la Société GOLDSCHMIDT | 4,4 | 6,6 |
| .Polyvinyl pyrrolidone vendue sous la dénomination "LUVISKOL K90 POWDER" par la société BASF | 6,0 | 6,0 |
| .Oxyde de fer noir | 6,0 | 6,0 |
| .Parahydroxybenzoate de méthyle | 0,1 | 0,1 |
| .Eau q.s.p. | 100,0 | 100,0 |

### EXEMPLE 8

| | |
|---|---|
| .Microdispersion de cire de carnauba vendue sous la dénomination "AQUACER 608" par la Société CERACHEMIE | 93,0 |
| .Oxyde de fer noir | 6,0 |
| .Hydroxyéthyl cellulose | 1,0 |
| Brillance de la composition : 24 | |

### EXEMPLE 9

| | |
|---|---|
| .Microdispersion de cires selon l'exemple C | 88,0 |
| .Oxyde de fer noir | 6,0 |
| .Gomme arabique | 2,0 |
| .Polyvinyl pyrrolidone | 4,0 |

### EXEMPLE 10

| | |
|---|---|
| .Microdispersion de cires selon l'exemple A | 74,0 |
| .Gomme arabique | 1,0 |
| .Silice colloïdale vendue sous la dénomination "COK 84" par la Société DEGUSSA | 4,0 |
| .Glycérine | 15,0 |
| .Oxyde de fer noir | 6,0 |

### EXEMPLES 11 à 14

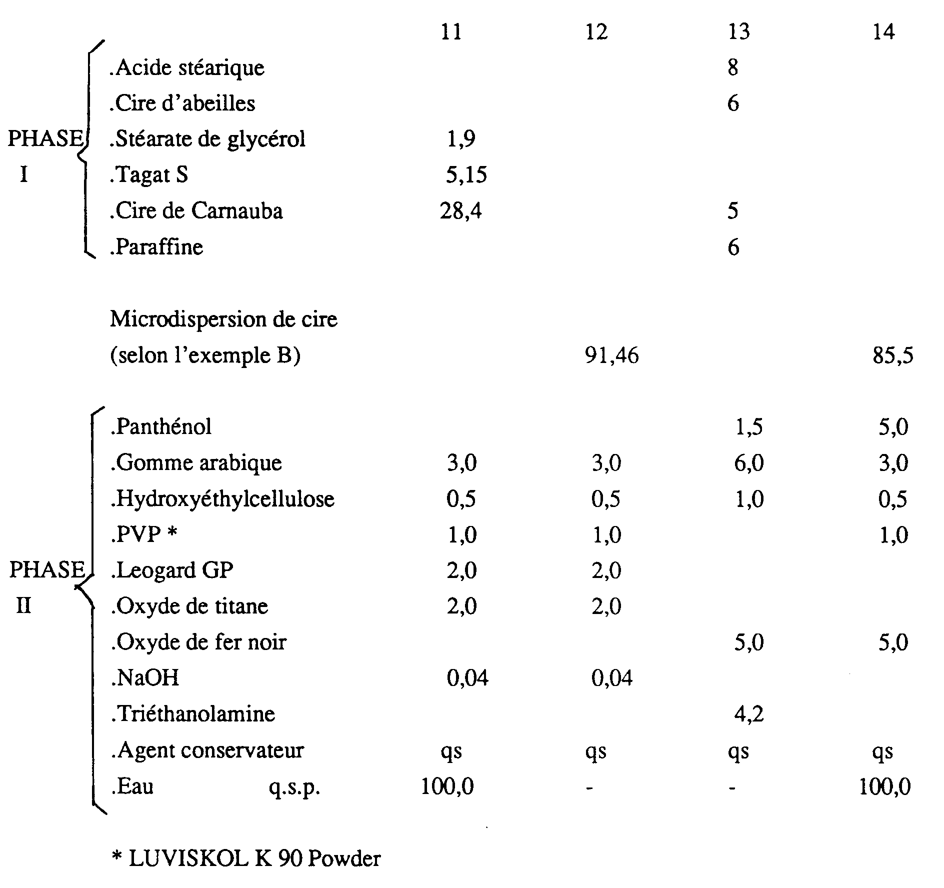

Les préparations 12 et 14 sont réalisées selon le mode opératoire décrit ci-dessus pour l'exemple 1. Les préparations 11 et 13 sont réalisées en mélangeant la phase I portée à 85°C. La phase II est préparée en portant l'eau à 85°C et en y ajoutant les polymères. L'émulsion est réalisée en ajoutant la phase II à la phase I à une température de 82°C. Puis l'oxyde de titane ou l'oxyde de fer est ajouté. La température est diminuée progressivement tout en conservant une agitation vigoureuse de la pâte.

### COMPARAISON DES EXEMPLES 11 ET 12

La préparation 12 appliquée sur les cils présente un aspect nettement plus lisse et plus brillant que la composition 11.

Les préparations 11 et 12 sont déposées sur une plaque de verre à l'aide d'un applicateur de façon à faire un dépôt homogène. Une fois secs, les films ainsi réalisés ont une épaisseur d'environ 100µm. Celle-ci correspond à l'épaisseur déposée en moyenne sur les cils lors du maquillage.

Le film de mascara 12 est très nettement plus brillant et plus lisse que le film du mascara 11.

L'état de surface a été étudié avec un profilomètre à laser. Cette étude a confirmé l'aspect très nettement plus lisse du film 12.

### COMPARAISON DES EXEMPLES 13 ET 14

Des cils ont été maquillés avec les formules des exemples 13 et 14 puis des photos en microscopie électronique des cils ont été réalisées.

La composition 13 (taille des grains de cire > 1 000nm) représente l'état de la technique tandis que la composition 14 représente une composition de cires microdispersées selon l'invention.

L'observation visuelle des résultats du maquillage a montré que le mascara 14 appliqué sur les cils présente un aspect bien plus lisse et régulier que le mascara 13.

Les photos en microscopie électronique ont confirmé ces observations.

### MODE OPERATOIRE DE FABRICATION DES COMPOSITIONS DES EXEMPLES 1 à 14, 21 et 22

### Mode opératoire I (Compositions 1 à 5, 8 et 9)

On opère par dilution de la microdispersion de cires de départ (2 étapes).

A température ambiante, le ou les polymères sont incorporés dans la microdispersion de cires sous agitation avec la quantité d'eau nécessaire (éventuellement) jusqu'à obtention d'une préparation homogène. Ensuite, on disperse les pigments.

La formulation obtenue peut être broyée.

La composition 5 est obtenue par addition des tensioactifs fluorés suivie de broyage.

### Mode opératoire II (Compositions 6 et 7)

La préparation de la composition de maquillage s'effectue en une étape à chaud.

Cires, tensioactifs et conservateurs sont fondus et mélangés ensemble à 90°C.

Les pigments sont dispersés dans la phase lipophile vers 90°C.

Le polymère est dissous dans l'eau à froid. La phase aqueuse est alors chauffée à 90°C et versée dans la phase liphophile sous agitation, tout en maintenant la température vers 90°C jusqu'à homogénéité. Puis la composition est refroidie et éventuellement passée au broyeur.

### Mode opératoire III (Composition 10)

A froid, les pigments et la silice sont dispersés dans la microdispersion de cires.

Le polymère et le(s) additif(s) hydrophile(s) sont ajoutés sous agitation douce, jusqu'à obtention d'une préparation homogène. La préparation peut être ensuite broyée.

### Mesure de la brillance

La préparation est étalée (100 à 300µm d'épaisseur) sur une plaque de verre. Après 24 heures de séchage, la brillance est mesurée avec un brillancemètre Microgloss (BYK) sous un angle de 85°.

### EXEMPLES N° 15 à 20

| | 15 | 16 | 17 | 18 | 19 | 20 |
|---|---|---|---|---|---|---|
| Microdispersion de cires selon | | | | | | |
| l'exemple B | 88,4 | 89,5 | 90,3 | 87,5 | 85,5 | |
| l'exemple D | | | | | | 86,2 |
| Huile de germe de blé | 1 | | | | | |
| Huile de silicone (PDMS) | | | | 0,5 | | |
| Huile de paraffine | | | | | 1,5 | |
| PVP | 2 | 1 | | 2,5 | | 1 |
| Luviquat FC905 (BASF) | | | | | 3 | |
| Leogard GP (AKZO) | | | 0,2 | 3 | | |
| Darvan 7 (VANDERBILT) | | | 2 | | | |
| Gomme arabique | 1,5 | 3 | | | | 5 |
| Hydroxyéthylcellulose | | 0,5 | 1,5 | | | 0,8 |
| Kérasol (CRODA) | | | | | 1,5 | |
| Carbopol 1342 (GOODRICH) | 0,6 | | | | | |
| Noir de carbone | | | 3 | | | |
| Oxyde de fer noir | 6 | 5 | | 1 | 1 | 5 |
| Oxyde de fer brun | | | | 4 | | |
| Bleu d'outremer | | 1 | | | 4 | 1 |
| Talc | | | | 1 | | |
| Orgasol 2002 Natcos (ATO) | | | 2 | | | |
| α-Bisabolol | | | | 0,5 | | |
| Panthénol | | | 1 | | 5 | 1 |
| Triéthanolamine | 0,5 | | | | | |
| Conservateurs | qs | qs | qs | qs | qs | qs |

Le mode opératoire est celui de l'exemple 1. Toute les compositions de ces exemples ont une viscosité comprise entre 4 et 25 Pa.s.

Toutes ces préparations appliquées sur les cils donnent un effet épaississant tout en leur conférant un aspect lisse et régulier très marquant. De plus, les exemples 16, 18 et 20 correspondent à des mascaras de brillance très élevée.

L'huile de silicone PDMS est celle vendue sous la dénomination DOW CORNING 200 Fluid par la Société DOW CORNING.

PVP : Polyvinyl pyrrolidone vendue sous le dénomination LUVISKOL K90 POWDER par la Société BASF.

LUVIQUAT FC 905 : copolymère de chlorure de méthyl vinylimidazolium et de vinylpyrrolidone (95/5), solution à 40 % de matière active dans l'eau vendue par la Société BASF.

LEOGARD GP : Hydroxyéthylcellulose réticulée à l'épichlorhydrine et quaternisée par la triméthylamine, vendue par la Société AKZO

DARVAN 7 : Polyméthacrylate de sodium, solution à 25% de matière active dans l'eau vendue par la Société VANDERBILT.

KERASOL : Hydrolysat de kératine stabilisé, vendu par la Société CRODA.

CARBOPOL 1342 : Polymère réticulé acrylate/C₁₀C₃₀ alkyl acrylate vendu par la société GOODRICH.

ORGASOL 2002 NATCOS : Nylon 12 (nom CTFA), poudre vendue par la Société ATO.

### EXEMPLE 21

| | |
|---|---|
| .Microdispersion de l'exemple E | 90 g |
| .Hydroxyéthylcellulose | 1 g |
| .Gomme arabique | 1,5 g |
| .Alcool polyvinylique vendu sous la dénomination "RHODOVIOL 4/125" par la Société RHONE POULENC | 0,5 g |
| .Oxyde de fer noir | 5,0 g |
| .Bleu Outremer | 2,0 g |
| .Conservateurs qs | |

### EXEMPLE 22

Cette composition est analogue à celle de l'exemple 21, mais en utilisant la microdispersion de l'exemple F à la place de celle de l'exemple E.

## Revendications

1. Composition pour le maquillage des yeux, caractérisée par le fait qu'elle comprend une dispersion aqueuse de particules de cire, au moins un polymère filmogène hydrosoluble et des pigments, et que ladite dispersion est une microdispersion aqueuse d'au moins une cire, ladite composition ayant une viscosité de 2,5 Pa.s à 35 Pa.s, à 25°C.

2. Composition selon la revendication 1, caractérisée par le fait que ladite composition a une viscosité comprise entre 3,5 et 25 Pa.s, à 25°C.

3. Composition selon la revendication 1 ou 2, caractérisée par le fait que les particules de cire ont des dimensions inférieures à 1 000nm.

4. Composition selon la revendication précédente, caractérisée par le fait que lesdites dimensions sont inférieures à 500nm.

5. Composition selon l'une quelconque des revendications 1 à 4, caractérisée par le fait qu'elle contient de 0,1 à 40% en poids, et en particulier de 5 à 30% de particules de cire.

6. Composition selon l'une quelconque des revendications précédentes, caractérisée par le fait que la cire ou le mélange de cire a un point de fusion compris entre 50 et 100°C.

7. Composition selon l'une quelconque des revendications précédentes, caractérisée par le fait que la cire ou le mélange de cires contient au moins une cire végétale choisie parmi la cire de Carnauba, la cire de Candellila et la cire d'Alfa.

8. Composition selon la revendication précédente, caractérisée par le fait que la cire ou le mélange de cires contient au moins 20% en poids et en particulier au moins 50% en poids de ladite cire végétale, par rapport au poids total de cire.

9. Composition selon l'une quelconque des revendications 7 et 8, caractérisée par le fait que la cire ou le mélange de cires contient au moins une autre cire et/ou au moins une huile, étant entendu que le mélange de cires et éventuellement d'huile a un point de fusion finissante supérieur à 50°C.

10. Composition selon la revendication précédente, caractérisée par le fait que lesdites autres huiles ou cires représentent au plus 30%, et en particulier au plus 10% en poids par rapport au poids de cires.

11. Composition selon l'une quelconque des revendications précédentes, caractérisée par le fait que la microdispersion de cire contient au moins un ingrédient actif liposoluble représentant au maximum 30% et en particulier au maximum 10% du poids des cires.

12. Composition selon l'une quelconque des revendications précédentes, caractérisée par le fait qu'elle contient au moins un tensioactif, dans une proportion comprise entre 0,01 et 25%, en particulier entre 0,1 et 10% en poids, par rapport au poids total de la composition.

13. Composition selon l'une quelconque des revendications précédentes, caractérisée par le fait qu'elle contient de 0.1 à 25%, et en particulier de 0.2 à 15% en poids dudit polymère filmogène.

14. Composition selon l'une quelconque des revendications précédentes, carctérisée par le fait qu'elle contient au moins un agent épaississant et/ou un agent de texture en quantité suffisante pour ajuster la viscosité à la valeur désirée.

15. Composition selon l'une quelconque des revendications précédentes, caractérisée par le fait que le polymère filmogène est choisi parmi l'hydroxyéthyl cellulose, la gomme arabique, la polyvinylpyrrolidone, les dérivés de cellulose cationiques, le polyméthacrylate de sodium, et les hydrolysats de kératine.

16. Composition selon l'une quelconque des revendications précédentes, caractérisée par le fait qu'elle contient de 0.1 à 20% en poids d'au moins un pigment et/ou une charge particulaire.

17. Utilisation d'une microdispersion aqueuse de cire en association avec au moins un polymère filmogène hydrosoluble et des pigments, dans la préparation d'une composition selon la revendication 1 pour le maquillage des yeux.

18. Utilisation selon la revendication précédente, caractérisée par le fait que ladite composition est telle que définie dans l'une quelconque des revendications 2 à 16.

19. Procédé de maquillage des yeux, caractérisé par le fait que l'on applique sur les cils et/ou sur le bord des paupières, une composition telle que définie dans l'une quelconque des revendications 1 à 16.

## Claims

1. Make-up composition for the eyes, characterized in that it comprises an aqueous dispersion of wax particles, at least one water-soluble film-forming polymer and pigments, and in that the said dispersion is an aqueous microdispersion of at least one wax, the said composition having a viscosity of 2.5 Pa s to 35 Pa s, at 25°C.

2. Composition according to Claim 1, characterized in that the said composition has a viscosity between 3.5 and 25 Pa s, at 25°C.

3. Composition according to Claim 1 or 2, characterized in that the wax particles are smaller than 1000 nm in size.

4. Composition according to the preceding claim, characterized in that the said sizes are smaller than 500 nm.

5. Composition according to any one of Claims 1 to 4, characterized in that it contains from 0.1 to 40 % by weight, and in particular from 5 to 30 %, of wax particles.

6. Composition according to any one of the preceding claims, characterized in that the wax or the wax mixture has a melting point between 50 and 100°C.

7. Composition according to any one of the preceding claims, characterized in that the wax or the mixture of waxes contains at least one plant wax chosen from carnauba wax, candelilla wax and esparto grass wax.

8. Composition according to the preceding claim, characterized in that the wax or the mixture of waxes contains at least 20 % by weight, and in particular at least 50 % by weight, of the said plant wax relative to the total weight of wax.

9. Composition according to either of Claims 7 and 8, characterized in that the wax or the mixture of waxes contains at least one other wax and/or at least one oil, it being understood that the mixture of waxes and possibly of oil has an end melting point above 50°C.

10. Composition according to the preceding claim, characterized in that the said other oils or waxes represent not more than 30 %, and in particular not more than 10 %, by weight relative to the weight of waxes.

11. Composition according to any one of the preceding claims, characterized in that the microdispersion of wax contains at least one liposoluble active ingredient representing a maximum of 30 %, and in particular a maximum of 10 %, of the weight of the waxes.

12. Composition according to any one of the preceding claims, characterized in that it contains at least one surfactant, in a proportion between 0.01 and 25 %, and in particular between 0.1 and 10 %, by weight relative to the total weight of the composition.

13. Composition according to any one of the preceding claims, characterized in that it contains from 0.1 to 25 %, and in particular from 0.2 to 15 %, by weight of the said film-forming polymer.

14. Composition according to any one of the preceding claims, characterized in that it contains at least one thickening agent and/or one texturing agent in an amount which is sufficient to adjust the viscosity to the desired value.

15. Composition according to any one of the preceding claims, characterized in that the film-forming polymer is chosen from hydroxyethyl cellulose, gum arabic, polyvinylpyrrolidone, cationic cellulose derivatives, sodium polymethacrylate and keratin hydrolysates.

16. Composition according to any one of the preceding claims, characterized in that it contains from 0.1 to 20 % by weight of at least one pigment and/or one particulate filler.

17. Use of an aqueous microdispersion of wax in combination with at least one water-soluble film-forming polymer and pigments, in the preparation of a composition, according to Claim 1, for making up the eyes.

18. Use according to the preceding claim, characterized in that the said composition is as defined in any one of Claims 2 to 16.

19. Process for making up the eyes, characterized in that a composition as defined in any one of Claims 1 to 16 is applied to the eyelashes and/or to the edge of the eyelids.

## Patentansprüche

1. Zubereitung zum Schminken der Augen, dadurch gekennzeichnet, daß sie eine wäßrige Dispersion aus Wachsteilchen, mindestens einem wasserlöslichen bildenden Polymer und Pigmente enthält, wobei diese Dispersion in Form einer wäßrigen Mikrodispersion aus mindestens einem Wachs vorliegt und die Zubereitung eine Viskosität von 3,5 Pa . s bis 25 Pa . s bei 25 °C aufweist.

2. Zubereitung nach Anspruch 1, dadurch gekennzeichnet, daß die Zubereitung eine Viskosität zwischen 3,5 und 25 Pa . s bei 25 °C aufweist.

3. Zubereitung nach Anspruch 1 oder 2, dadurch gekennzeichnet, daß die Wachsteilchen einen Durchmesser von weniger als 1000 nm besitzen.

4. Zubereitung nach dem vorstehenden Anspruch, dadurch gekennzeichnet, daß der Durchmesser jeweils weniger als 500 nm beträgt

5. Zubereitung nach einem der Ansprüche 1 bis 4, dadurch gekennzeichnet, daß sie 0,1 bis 40 Gew.%, insbesondere 5 bis 30 Gew.% Wachs enthält.

6. Zubereitung nach einem der vorstehenden Ansprüche, dadurch gekennzeichnet, daß das Wachs oder die Wachsmischung einen Schmelzpunkt zwischen 50 ° und 100 °C aufweist.

7. Zubereitung nach einem der vorstehenden Ansprüche, dadurch gekennzeichnet, daß das Wachs oder die Wachsmischung mindestens ein pflanzliches Wachs enthält, das aus Carnaubawachs, Candellilawachs und Alfawachs ausgewählt ist.

8. Zubereitung nach dem vorstehenden Anspruch, dadurch gekennzeichnet, daß das Wachs oder die Wachsmischung mindestens 20 Gew.% und insbesondere mindestens 50 Gew.% Pflanzenwachs in bezug auf das Gesamtgewicht an Wachs enthält.

9. Zubereitung nach einem der Ansprüche 7 und 8, dadurch gekennzeichnet, daß das Wachs oder die Wachsmischung mindestens noch ein weiteres Wachs und/oder mindestens ein Öl enthält, wobei in diesem Falle die Wachsmischung und gegebenenfalls das Öl einen Endschmelzpunkt von mehr als 50 °C aufweisen.

10. Zubereitung nach dem vorangegangenen Anspruch, dadurch gekennzeichnet, daß die weiteren Öle oder Wachse bis zu 30 Gew.% und insbesondere bis zu 10 Gew.% in bezug auf das Wachsgewicht ausmachen.

11. Zubereitung nach einem der vorstehenden Ansprüche, dadurch gekennzeichnet, daß die Wachsmikrodispersion mindestens einen fettlöslichen Wirkstoff enthält, der in einer maximalen Menge von 30 Gew.% und insbesondere in einer maximalen Menge von 10 Gew.% an Wachsen vorliegen.

12. Zubereitung nach einem der vorstehenden Ansprüche, dadurch gekennzeichnet, daß sie mindestens ein Tensid in einer Menge enthält, die zwischen 0,01 und 25 Gew.%, insbesondere zwischen 0,1 und 10 Gew.% in bezug auf das Gesamtgewicht der Zusammensetzung liegt.

13. Zubereitung nach einem der vorstehenden Ansprüche, dadurch gekennzeichnet, daß sie 0,1 bis 25 Gew.% und insbesondere 0,2 bis 15 Gew.% an filmbildendem Polymer enthält.

14. Zubereitung nach einem der vorstehenden Ansprüche, dadurch gekennzeichnet, daß sie mindestens ein Verdickungsmittel und/oder ein Texturmittel in einer Menge enthält, die ausreicht, die Viskosität auf den gewünschten Wert einzustellen.

15. Zubereitung nach einem der vorstehenden Ansprüche, dadurch gekennzeichnet, daß das filmbildene Polymer aus Hydroxyethylcellulose, Gummi arabicum, Polyvinylpyrrolidon, kationischen Cellulosederivaten, Natriumpolymethacrylat und Keratinhydrolysaten ausgewählt ist.

16. Zubereitung nach einem der vorstehenden Ansprüche, dadurch gekennzeichnet, daß sie 0,1 bis 20 Gew.% mindestens eines Pigments und/oder eines teilchenförmigen Trägers enthält.

17. Verwendung einer wäßrigen Wachsmikrodispersion in Kombination mit mindestens einem wasserlöslichen filmbildenden Polymer und Pigmenten zur Herstellung einer Zubereitung nach Anspruch 1 für das Schminken der Augen.

18. Verwendung gemäß vorstehendem Anspruch, dadurch gekennzeichnet, daß die Zubereitung nach einem der Ansprüche 2 bis 16 zusammengesetzt ist.

19. Verfahren zum Schminken der Augen, dadurch gekennzeichnet, daß auf den Wimpern und/oder dem Rand der Augenbrauen eine Zubereitung appliziert wird, wie sie nach einem der Ansprüche 1 bis 16 zusammengesetzt ist.
